# EUROPEAN PATENT SPECIFICATION

(11) **EP 1 013 240 B1**
(45) Date of publication and mention of the grant of the patent: **26.11.2003**
(21) Application number: 99310204.5
(22) Date of filing: 16.12.1999
(51) Int. Cl.: A61F 2/30, A61F 2/34, A61B 17/80

(54) **Prosthetic acetabulum fixing plate**
Prothetische Acetabulumbefestigungsplatte
Plaque de fixation prothétique pour l'acétabule

(30) Priority: 17.12.1998 GB 9827879
(43) Date of publication of application: 28.06.2000
(73) Proprietor: BENOIST GIRARD SAS, 14200 Hérouville-Saint-Clair (FR)
(72) Inventor: Ling, Prof. Robin Sydney Macwood, Dartmouth, Devon TQ6 0HB (GB); Gie, Graham Allan, Yeoford, Devon EX17 5EZ (GB); Timperley, Andrew John, St. Leonard's, Exeter EX2 4PA (GB); Storer, John Andrew, 14250 Jouaye Mondaye, Bayeux (FR)
(74) Representative: Bridge-Butler, Alan James

(56) References cited:
- EP-A- 0 328 847
- EP-A- 0 834 294
- DE-A- 2 837 400
- DE-B- 2 410 057
- FR-A- 2 056 934
- FR-A- 2 633 823
- GB-A- 1 430 071
- US-A- 3 866 248

## Description

This invention relates to the use of prosthetic ball joint cups in the surgical procedures to renew skeletal joints. During these procedures it has been noted that any discontinuity in the rim or wall of the acetabulum of the innominate bone for example the acetabular notch beneath the transverse ligament is instrumental in causing problems where the fixing cement used to bond the ball joint cup to the surface of the acetabulum is often lost through the discontinuity.

In order to prevent this loss of cement a system is hereby proposed where a fixing plate is placed inside the acetabulum, blocking the discontinuity and allowing the ball joint cup to be pushed into place without loss of cement.

GB-A-1 430 071 shows a cement restrictor which comprises a flanged hollow member which can be located in an acetabulum. The specification does not describe precisely how the restrictor is located in the acetabulum and the walls of the restrictor are perforated so that they provide no restriction to the loss of cement through any discontinuity in the wall of the acetabulum.

FR-A-2 633 823 also shows a plate for fixing in the acetabulum which is hemispherical with an opening at the bottom. Openings are also provided around the sides and the insert is held in place by screws. The specification refers to cement passing through the openings (23). Thus, this construction does not prevent loss of cement through discontinuities in the rim or wall of the acetabulum.

US-A-3 866 248 shows a cement restrictor for use in a hip replacement operation. The device comprises a domed portion with a projecting flange and the wall is perforated with shaped openings or slots through which cement can pass. Again, with this construction there is no provision for preventing fixing cement being lost through any discontinuity in the wall or rim of the acetabulum.

FR-A-2 056 934 describes a cement restrictor for use in an acetabulum and is designed for preventing cement entering the base of the acetabular cavity (ossa) when an acetabular cup is implanted. This cement restrictor does not however act to prevent the escape of cement through any discontinuity in the rim or wall of the acetabulum.

DE-A-28 37 400 shows a cement restrictor made from metal gauze and which is located at the base of the socket. This restrictor is ineffective with regard to loss of cement through the rim or wall of the acetabulum and is essentially for use in the ossa.

According to the present invention a prosthetic acetabulum fixing plate for use with a fixing cement comprises a component (1) to be located into the acetabulum (3) prior to the insertion of a prosthetic acetabular cup characterised by a closure wall (7) adapted to close the wall or rim (6) of the acetabulum (3) across any discontinuity (4) therein, and location means (8) for locating said closure wall (7) in place and which engage over the rim (10) at each side of the discontinuity (4).

The fixing plate can be supplied in various sizes and arrangements, the correct plate being chosen by the surgeon after close examination of X-ray photographs of the patient and through the use of a template which is used to determine the size and arrangement of the discontinuity and thus the correct size and arrangement of the plate to be used.

The plate (1) can be made from any suitable material but is preferably made from a synthetic plastic material for example polymethyl methacrylate, (P.M.M.A.).

Preferably the fixing plate (1) also has a lining portion adapted to extend across the depressed portion of the acetabulum, (the fossa (5)).

This lining portion can have apertures (2) to enable fixing cement to extend through it.

The plate can be used after a filling material such as bone graft is used to fill the depression in the inner surface of the acetabulum, said component then being placed over the filling material.

The plate can then become a continuation of the substantially continuously circular nature of the outer rim of the acetabulum.

An inwardly projecting ridge or ledge (11) can be provided to receive and locate an acetabulum cup with a disposable flange or a fixed flange or indeed without a flange to provide location and depth defining means for the cup.

The plate can be dimensioned in varying size dependant on the patient.

The location means (8) may conveniently comprise a clip (8) adapted to extend over the rim (10) of the acetabulum adjacent to any discontinuity (4).

The invention can be performed in various ways but one embodiment will now be described by way of example and with reference to the accompanying drawings in which:
Figure 1 is a partial front elevation of the innominate bone and the prosthetic acetabulum fixing plate according to the invention;
Figure 2 is a partial isometric view of the innominate bone and the prosthetic fixing plate;
Figure 3 is a partial isometric cross section of the innominate bone and a second embodiment of the prosthetic fixing plate;
Figure 4 is a plan view of the second embodiment of the prosthetic acetabulum fixing plate as shown in figure 3; and,
Figure 5 is an isometric view of the prosthetic acetabulum fixing plate shown in Figure 4.

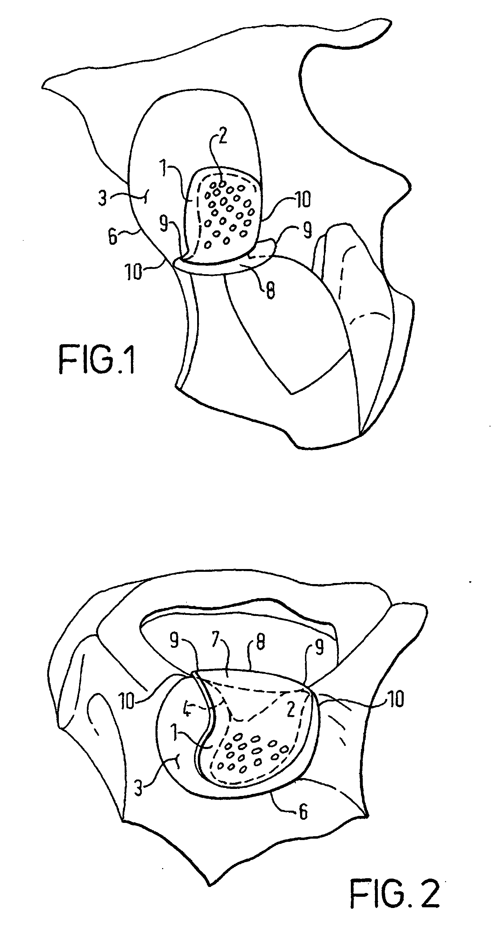

As shown in figures 1-5 a prosthetic acetabulum fixing plate according to the invention comprises a plate (1) having a lining portion which has apertures (2) which is dished to allow it to be located contiguously with the inner surface of the acetabulum (3) covering the discontinuity (4) of the rim (6) of the acetabulum and the depression, or fossa (5) in the inner surface of the acetabulum (3) and having a closure wall portion (7) which extends across the discontinuity (4)

In addition to said plate (1) a corresponding amount of bone graft (not shown) is placed into the fossa (5) prior to said plate (1) being positioned.

With said plate (1) located in position a corresponding amount of fixing cement (not shown) is placed within the acetabulum (3) and a prosthetic ball joint cup (not shown) is pushed into place. When said fixing cement is pressurised it passes through the apertures (2) in the plate and mixes with the bone graft (not shown) in the depression (5) of the acetabulum thus bonding said prosthetic fixing plate (1) and said cup into position.

To assist in locating the plate the closure wall (7) is extended to form a reentrant clip (8) each spaced apart outer end (9) of which engages over the rim portion (10) of the acetabulum (3) at each side of the discontinuity (4). This clip (8) enables the plate (1) to be firmly located prior to insertion of cement (not shown) and the acetabulum cup which is to be secured.

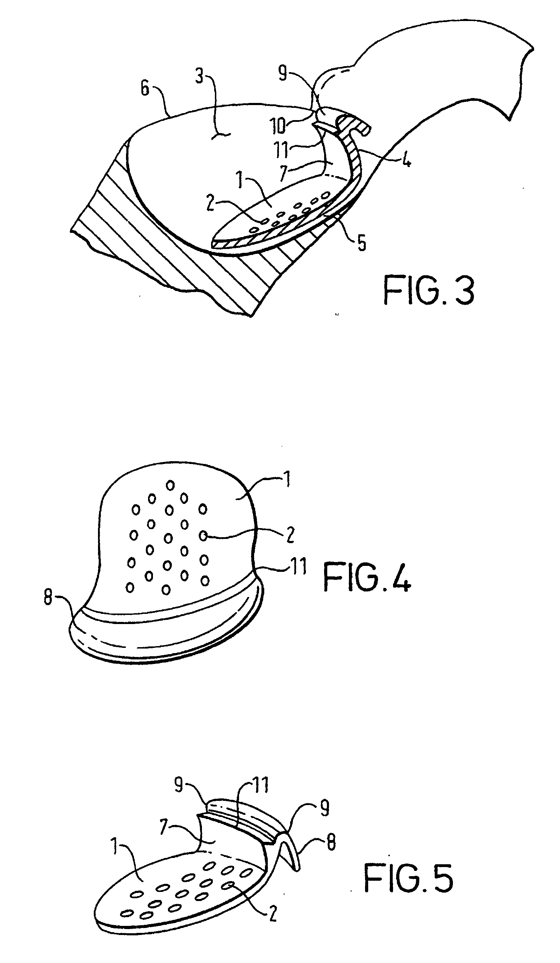

With reference to the embodiment shown in figures 3-5 it can be seen that a ridge or ledge (11) can be included in the surface of the closure wall (7) to provide a location and depth defining means for a prosthetic acetabular cup (not shown) provided with a flange, or a rim, for example as described and shown in EP 098309156.8, which will be inserted into the acetabulum after the fixing plate (1) has been properly located.

## Claims

1. A prosthetic acetabulum fixing plate for use with a fixing cement comprising a component (1) to be located into the acetabulum (3) prior to the insertion of a prosthetic acetabular cup **characterised by** a closure wall (7) adapted to close the wall or rim (6) of the acetabulum (3) across any discontinuity (4) therein, and location means (8) for locating said closure wall (7) in place and which engage over the rim (10) at each side of the discontinuity (4).

2. A prosthetic acetabulum fixing plate as claimed in claim 1 **characterised in that** said closure wall (7) is made from synthetic plastics material.

3. A prosthetic acetabulum fixing plate as claimed in claim 2 **characterised in that** said synthetic plastics material is polymethyl methacrylate, (P.M.M.A.).

4. A prosthetic acetabulum fixing plate as claimed in claims 1 to 3 **characterised by** a lining portion adapted to extend across the depressed portion of the acetabulum, (the fossa (5)).

5. A prosthetic acetabulum fixing plate as claimed in claim 4 **characterised in that** said lining portion has apertures (2) to enable fixing cement to extend through it.

6. A prosthetic acetabulum fixing plate as claimed in any one of the preceding claims 1 to 5 **characterised by** an inwardly projecting ridge or ledge (11) to receive and locate an acetabulum cup with a disposable flange, fixed flange or no flange to provide location and depth defining means for the cup.

7. A prosthetic acetabulum fixing plate as claimed in 6 **characterised in that** said projecting ridge or ledge (11) is adapted to project into the acetabulum at a point below the plane of the opening of the acetabulum.

8. A prosthetic acetabulum fixing plate as claimed in claims 1 to 7 **characterised in that** the location means (8) comprise a clip (8) adapted to extend over the rim (10) of the acetabulum adjacent to any discontinuity (4).

## Patentansprüche

1. Prothetische Acetabulumbefestigungsplatte zur Verwendung mit einem Befestigungszement, umfassend eine vor dem Einsetzen einer prothetischen Hüftgelenkspfanne in das Acetabulum zu positionierende Komponente (1), **gekennzeichnet durch** eine Verschlusswand (7), die angepasst ist, um die Wand oder den Rand (6) des Acetabulums (3) über irgendeine darin befindliche Unterbrechung (4) hinweg zu verschließen, sowie Positioniereinrichtungen (8), um die Verschlusswand (7) an Ort und Stelle zu positionieren und welche an jeder Seite der Unterbrechung (4) über dem Rand (10) in Eingriff treten.

2. Prothetische Acetabulumbefestigungsplatte nach Anspruch 1, **dadurch gekennzeichnet, dass** die Verschlusswand (7) aus synthetischem Kunststoffmaterial hergestellt ist.

3. Prothetische Acetabulumbefestigungsplatte nach Anspruch 2, **dadurch gekennzeichnet, dass** das synthetische Kunststoffmaterial Polymethylmethacrylat (PMMA) ist.

4. Prothetische Acetabulumbefestigungsplatte nach den Ansprüchen 1 bis 3, **gekennzeichnet durch** einen Auskleidungsteil, der angepasst ist, um sich über den vertieften Teil des Acetabulums (die Fossa (5)) zu erstrecken.

5. Prothetische Acetabulumbefestigungsplatte nach Anspruch 4, **dadurch gekennzeichnet, dass** der Auskleidungsteil Öffnungen (2) aufweist, um es zu ermöglichen, dass sich Befestigungszement durch ihn hindurch erstreckt.

6. Prothetische Acetabulumbefestigungsplatte nach einem der vorangehenden Ansprüche 1 bis 5, **gekennzeichnet durch** eine nach innen überstehende Rippe oder Leiste (11), um eine Hüftgelenkspfanne mit einem Einwegflansch, einem festen Flansch oder ohne Flansch aufzunehmen und zu positionieren, um für die Pfanne Positionierungs- und Tiefenfestlegungseinrichtungen bereitzustellen.

7. Prothetische Acetabulumbefestigungsplatte nach Anspruch 6, **dadurch gekennzeichnet, dass** die überstehende Rippe oder Leiste (11) angepasst ist, um an einem Punkt unterhalb der Ebene der Öffnung des Acetabulums in das Acetabulum zu ragen.

8. Prothetische Acetabulumbefestigungsplatte nach den Ansprüchen 1 bis 7, **dadurch gekennzeichnet, dass** die Positioniereinrichtungen (8) einen Halter (8) umfassen, der angepasst ist, um sich benachbart von irgendeiner Unterbrechung (4) über den Rand (10) des Acetabulums zu erstrecken.

## Revendications

1. Plaque de fixation de prothèse cotyloïdienne destinée à être utilisée avec un ciment de fixation, comprenant un composant (1) devant être disposé dans le cotyle (3) ouvrant l'insertion d'une coupelle cotyloïdienne prothétique, **caractérisée par** une paroi de fermeture (7) apte à fermer la paroi ou bordure (6) du cotyle (3) à travers toute discontinuité (4) à l'intérieur, et des moyens de positionnement (8) pour positionner ladite paroi de fermeture (7) en place et qui s'engagent sur la bordure (10) de chaque côté de la discontinuité (4).

2. Plaque de fixation de prothèse cotyloïdienne selon la revendication 1, **caractérisée en ce que** ladite paroi de fermeture (7) est en matière plastique.

3. Plaque de fixation de prothèse cotyloïdienne selon la revendication 2, **caractérisée en ce que** ladite matière plastique est du polyméthacrylate de méthyle (PMMA).

4. Plaque de fixation de prothèse cotyloïdienne selon les revendications 1 à 3, **caractérisée par** une partie de revêtement apte à s'étendre à travers la partie renfoncée du cotyle (la fosse (5)).

5. Plaque de fixation de prothèse cotyloïdienne selon la revendication 4, **caractérisée en ce que** ladite partie de revêtement a des ouvertures (2) pour permettre à du ciment de fixation de la traverser.

6. Plaque de fixation de prothèse cotyloïdienne selon l'une quelconque des revendications précédentes 1 à 5, **caractérisée par** un bord ou corniche (11) en saillie vers l'intérieur pour recevoir et positionner une coupelle cotyloïdienne dans un rebord jetable, un rebord fixe ou pas de rebord, pour fournir des moyens de positionnement et de définition de profondeur pour la coupelle.

7. Plaque de fixation de prothèse cotyloïdienne selon la revendication 6, **caractérisée en ce que** ledit bord ou corniche en saillie (11) est apte à faire saillie dans le cotyle en un point sous le plan de l'ouverture du cotyle.

8. Plaque de fixation de prothèse cotyloïdienne selon les revendications 1 à 7, **caractérisée en ce que** les moyens de positionnement (8) comportent une attache (8) apte à s'étendre par-dessus la bordure (10) du cotyle au voisinage de toute discontinuité (4).
